(19) Europäisches Patentamt
European Patent Office
Office européen des brevets

(11) EP 3 925 595 A1

# (12) EUROPEAN PATENT APPLICATION

(43) Date of publication:
**22.12.2021 Bulletin 2021/51**

(51) Int Cl.:
***A61K 9/00*** *(2006.01)*

(21) Application number: **20180465.5**

(22) Date of filing: **17.06.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **AiCuris GmbH & Co. KG**
**42117 Wuppertal (DE)**

(72) Inventor: **REDMER, Jessica**
**41199 Mönchengladbach (DE)**

(74) Representative: **Arth, Hans-Lothar**
**ABK Patent Attorneys**
**Jasminweg 9**
**14052 Berlin (DE)**

## (54) OPHTHALMIC FORMULATION COMPRISING N-[5-(AMINOSULFONYL)-4-METHYL-1,3-THIAZOL-2-YL]-N-METHYL-2-[4-(2-PYRIDINYL)PHENYL] ACETAMIDE HEMIHYDRATE

(57) The present invention relates to an ophthalmic formulation comprising N-[5-(aminosulfonyl)-4-methyl-1,3-thiazol-2-yl]-N-methyl-2-[4-(2-pyridinyl)phenyl]acetamide hemihydrate

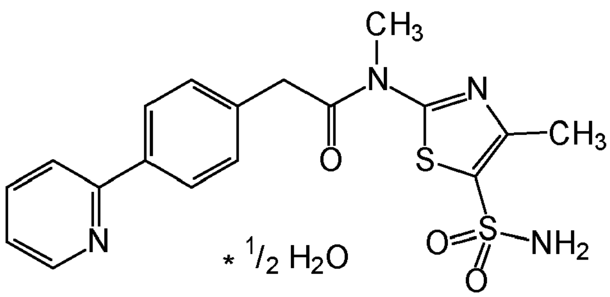

polyethylene glycol, and an artificial tears solution as well as to a method for preparing such an ophthalmic formulation.

EP 3 925 595 A1

## Description

The present invention relates to an ophthalmic formulation comprising N-[5-(aminosulfonyl)-4-methyl-1,3-thiazol-2-yl]-N-methyl-2-[4-(2-pyridinyl)phenyl]acetamide hemihydrate, polyethylene glycol, and an artificial tears solution as well as to a method for preparing such ophthalmic formulation.

## Background of the invention

Synthesis of N-[5-(aminosulfonyl)-4-methyl-1,3-thiazol-2-yl]-N-methyl-2-[4-(2-pyridinyl)phenyl]acetamide is known from WO 01/47904 A1, and the use of acidic components including methanesulfonic acid for the formulation of tablets for the treatment of herpes simplex infections. Such tablets contain micronized N-[5-(aminosulfonyl)-4-methyl-1,3-thiazol-2-yl]-N-methyl-2-[4-(2-pyridinyl)phenyl]acetamide as disclosed by WO 2006/103011 A1.

Herpes infections can also occur in the eye and in case the eye herpes only affects the outermost layers of the eye, antiviral eye drops containing an antiviral agent such as Acyclovir can be used for treatment. However, cases of eye herpes are known where the herpes viruses are resistant against the available antiviral herpes agents.

N-[5-(aminosulfonyl)-4-methyl-1,3-thiazol-2-yl]-N-methyl-2-[4-(2-pyridinyl)phenyl]-acetamide is known as an effective antiviral herpes agent which can be administered even in cases where resistance of herpes viruses against other available antiviral herpes agents is observed.

Therefore, it is the objective of the present invention to provide an ophthalmic formulation containing the compound N-[5-(aminosulfonyl)-4-methyl-1,3-thiazol-2-yl]-N-methyl-2-[4-(2-pyridinyl)phenyl]acetamide, so that drug resistant herpes infections of the eye can be treated.

The objective of the present invention is solved by the teachings of the independent claims. Further advantageous features, aspects and details of the invention are evident from the dependent claims, the description, and the examples of the present application.

## Description of the invention

The present invention is directed to an ophthalmic formulation comprising:

a) N-[5-(aminosulfonyl)-4-methyl-1,3-thiazol-2-yl]-N-methyl-2-[4-(2-pyridinyl)phenyl]acetamide hemihydrate,
b) polyethylene glycol, and
c) artificial tears solution.

The compound N-[5-(aminosulfonyl)-4-methyl-1,3-thiazol-2-yl]-N-methyl-2-[4-(2-pyridinyl)phenyl]acetamide is also known under the international nonproprietory name (INN) Pritelivir.

The compound N-[5-(aminosulfonyl)-4-methyl-1,3-thiazol-2-yl]-N-methyl-2-[4-(2-pyridinyl)phenyl]acetamide hemihydrate is also named herein "Pritelivir free base hemihydrate" or just "Pritelivir hemihydrate". The term "free base" in the designation "Pritelivir free base hemihydrate" shall indicate that use of the free base is essential to the invention and that no salts of the compound Pritelivir shall be used under the conditions disclosed herein for the preparation of the ophthalmic formulation.

**Pritelivir free base hemihydrate** has the following chemical structure:

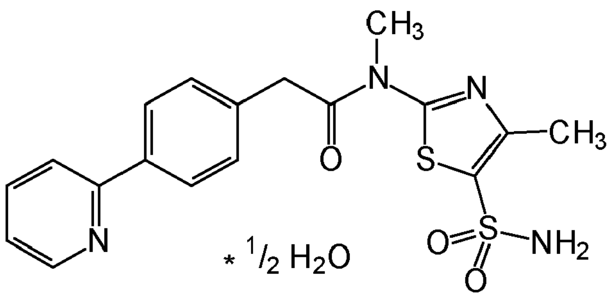

Several salts of Pritelivir such as the mesylate salt or the maleate salt of Pritelivir are known. However, in the course of developing the herein-described new ophthalmic formulation containing Pritelivir it was surprisingly found that no ophthalmic formulation could be provided on the basis of the tested salts of Pritelivir under the experimental conditions which were suited to provide the herein-described ophthalmic formulation containing Pritelivir free base hemihydrate as drug substance (DS).

Moreover, the observations made in the course of the development of the herein-described new ophthalmic formulation suggest that a salt formation of Pritelivir should be avoided during the preparation of the ophthalmic formulation. Consequently, the present application is directed to an ophthalmic formulation which is free from Pritelivir salts and which is useful for treatment of herpes infections of the eye and especially such herpes infections of the eye which already developed resistance against common antiviral herpes drugs.

**PEG: polyethylene glycol**

One essential component of the ophthalmic formulation of the present application is polyethylene glycol, abbreviated as PEG.

In compatibility studies and solubility studies a larger number of solvents and ingredients had been investigated but unexpectedly only PEG was found to be suitable and acceptable for an ophthalmic formulation of Pritelivir free base hemihydrate.

Polyethylene glycol has the following chemical structure

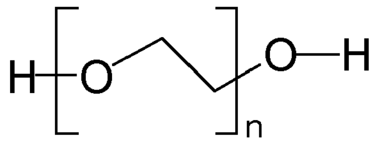

wherein n indicates the number of repeating units.

The chemical formula is $C_{2n}H_{4n+2}O_{n+1}$ and the density is 1.125 g/mL.

Other IUPAC names of PEG are poly(oxyethylene) or poly(ethylene oxide). PEG is also known under the trademarks Carbowax™, Kollisolv®, Kolliphor®, Polyglycol™ and the Ph. Eur. (Pharmacopoea Europaea) name Macrogol.

Several different polyethylene glycols such as PEG 200, PEG 300, PEG 400, PEG 600, PEG 1000, PEG 1500, PEG 2000, PEG 3000, PEG 4000, PEG 6000, PEG 8000 and so on are known.

The numbers in the names of the PEGs indicate their average molecular weight, e.g. a PEG with n = 9 has an average molecular weight of approximately 400 daltons, and is usually designated as PEG 400. Most PEGs include molecules with a distribution of molecular weights, i.e. they are polydisperse.

The size distribution can be characterized statistically by its weight average molecular weight (Mw) and its number average molecular weight (Mn), the ratio of which is called the polydispersity index (Mw/Mn). Mw and Mn can be measured by mass spectrometry.

It was found that polyethylene glycols having an average molecular weight in the range of from 200 g/mol to 400 g/mol are suitable for the present ophthalmic formulation. The use of PEG 400 is particularly preferred for the present ophthalmic formulation.

However, it is also possible to use mixtures of PEGs for the ophthalmic formulation. PEGs having an average molecular weight in the range of from 200 g/mol to 400 g/mol are at room temperature non-volatile liquids.

The terms "in the range of from *'value A'* to *'value B'*" and "an amount of from *'value A'* to *'value B'*" as used throughout the present application refers to a continuous group of possible values, wherein *'value A'* represents the lower end of said group and *'value B'* represents the upper end of said group. Values representing said lower and said upper ends are included in said group of possible values. For example, PEGs having an average molecular weight in the range of from 200 g/mol to 400 g/mol include PEGs having an average molecular weight of 200 g/mol and PEGs having an average molecular weight of 400 g/mol as well as PEGs having average molecular weights which are in between.

The term "room temperature" as used herein, is synonymous to the term "standard room temperature" and refers to a temperature in the range of from 19 °C to 26 °C. For example, PEGs which are "at room temperature non-volatile liquids" means that said PEGs are non-volatile liquids "at a temperature in the range of from 19 °C to 26 °C".

Therefore, it is also in accordance with the present invention to use mixtures of two, three or more PEGs in the ophthalmic formulation. For instance, mixtures of PEG 300 and PEG 400, or PEG 200 and PEG 400, or PEG 200 and PEG 300 and PEG 400, and so on.

The average molecular weight of all mixtures of PEGs used for the present ophthalmic formulation should be in the range of from 200 g/mol to 400 g/mol, preferably in the range of from 300 g/mol to 400 g/mol, and more preferably in the range of from 350 g/mol to 400 g/mol, and most preferably around 400 g/mol.

Therefore, the present application is also directed to an ophthalmic formulation comprising or consisting of:

a) N-[5-(aminosulfonyl)-4-methyl-1,3-thiazol-2-yl]-N-methyl-2-[4-(2-pyridinyl)phenyl]acetamide hemihydrate,
b) PEG 200, PEG 300, PEG 400, or a mixture of two or more of these PEGs, and

c) artificial tears solution.

Preferred is an ophthalmic formulation comprising or consisting of:

a) N-[5-(aminosulfonyl)-4-methyl-1,3-thiazol-2-yl]-N-methyl-2-[4-(2-pyridinyl)phenyl]acetamide hemihydrate,
b) PEG 200 and/or PEG 400, preferably PEG 400, and
c) artificial tears solution.

The PEG or the mixture of PEGs used in the present ophthalmic formulation is responsible for the solubility of Pritelivir free base hemihydrate, i.e. without the PEG or the mixture of PEGs the desired amount of Pritelivir free base hemihydrate cannot be dissolved in the ophthalmic formulation and precipitation occurs, reducing the amount of effective drug substance (i.e. Pritelivir free base hemihydrate) in the ophthalmic formulation so that no ophthalmic formulation with a defined amount of effective drug substance can be prepared.

Moreover, the PEG or the mixture of PEGs used in the present ophthalmic formulation is responsible for the viscosity of the ophthalmic formulation. Therefore, it is preferred that the PEG or the mixture of PEGs is selected such that the viscosity of the ophthalmic formulation is in the range of from 25 cps to 50 cps.

In addition to the PEG or the mixture of PEGs used for the present ophthalmic formulation also the selection of the artificial tears solution has an impact on the viscosity of the ophthalmic formulation. Taking into consideration that the artificial tears solution is normally the main component of the ophthalmic formulation, the viscosity of the ophthalmic formulation of the present application is essentially the same as the viscosity of the artificial tears solution. However, the PEG or the mixture of PEGs present in the ophthalmic formulation can change the viscosity of the artificial tears solution so that the ophthalmic formulation has preferably a viscosity which is identical or up to 20% different from that of the artificial tears solution.

This means that, if the artificial tears solution has for eample a viscosity of 40 cps, the ophthalmic formulation of the present application has preferably a viscosity in the range of from 32 cps to 48 cps, and has more preferably a viscosity close to 40 cps.

In general, the ophthalmic formulation of the present invention should have a viscosity in the range of from 10 cps to 100 cps, preferably in the range of from 15 cps to 80 cps, more preferably in the range of from 20 cps to 60 cps, still more preferably in the range of from 25 cps to 50 cps, and still more preferably in the range of from 30 cps to 40 cps.

Many everyday fluids have viscosities between 0.5 and 1000 cP. For instance, water has a viscosity of 1 cp. The lacrimal fluid has a viscosity in the range of from 1.0 cp to 6.0. Ophthalmic formulations with a viscosity up to 20 cp are well tolerated. However, in order to ensure that the ophthalmic formulation remains sufficiently long in the eye, viscosities between 25 cp and 50 cp are preferred and result normally only in an increased reflex regarding the tearing and blinking of the treated eye.

The viscosity of a solution is given in poise units. The poise is the unit of the dynamic viscosity (i.e., the absolute viscosity). The unit centipoise (cp or the plural cps) is equal to 0.01 poise. The SI unit of the viscosity is Pascal-second (Pa * s), equivalent to Newton-second per square meter (N * s * $m^{-2}$).

$$1000\ \mathrm{cp} = 10\ \mathrm{p} = 1\ \mathrm{Pa} * \mathrm{s} = 1\ \mathrm{N} * \mathrm{s} * \mathrm{m}^{-2} = 1\ \mathrm{kg} * \mathrm{m}^{-1} * \mathrm{s}^{-1}$$

Thus, in order to obtain the desired viscosity, it is also possible to add a viscosity enhancer to the inventive ophthalmic formulation.

Suitable viscosity enhancers are hydroxyethylcellulose, polyvinyl alcohol, hydroxypropylmethylcellulose, methylcellulose, and polyvinylpyrrolidone. The viscosity enhancer should not be used in the ophthalmic formulation in an amount exceeding 1.0 weight%.

Therefore, the present application is also directed to an ophthalmic formulation comprising or consisting of:

a) N-[5-(aminosulfonyl)-4-methyl-1,3-thiazol-2-yl]-N-methyl-2-[4-(2-pyridinyl)phenyl]acetamide hemihydrate,
b) polyethylene glycol, and
c) artificial tears solution,

wherein the ophthalmic formulation has a viscosity in the range of from 25 cp to 50 cp.

Preferred is an ophthalmic formulation comprising or consisting of:

a) N-[5-(aminosulfonyl)-4-methyl-1,3-thiazol-2-yl]-N-methyl-2-[4-(2-pyridinyl)phenyl]acetamide hemihydrate,
b) PEG 200, PEG 300, PEG 400, or a mixture of two or more of these PEGs, and
c) artificial tears solution,

wherein the ophthalmic formulation has a viscosity in the range of from 25 cp to 50 cp.

Also preferred is an ophthalmic formulation comprising or consisting of:

a) N-[5-(aminosulfonyl)-4-methyl-1,3-thiazol-2-yl]-N-methyl-2-[4-(2-pyridinyl)phenyl]acetamide hemihydrate,
b) PEG 200 and/or PEG 400, preferably PEG 400, and
c) artificial tears solution,

wherein the ophthalmic formulation has a viscosity in the range of from 25 cp to 50 cp.

The pH value of the ophthalmic formulation of the present application should be in the range of from 4.0 to 8.5, preferably in the range of from 4.2 to 8.0, more preferably in the range of from 4.5 to 7.5, still more preferably in the range of from 5.0 to 7.0, still more preferably in the range of from 5.5 to 6.5, and still more preferably in the range of from 5.7 to 6.3.

Although the lacrimal fluid has a physiological pH value of about 7.4, the experiments revealed herein demonstrated that pH values below 7.0 are beneficial over pH values above pH 7.0.

Preferably, the pH value of the ophthalmic formulation is adjusted by the addition of aqueous hydrochlorid acid solution, preferably by the addition of a 0.1M $HCl_{aq}$ solution.

For pH adjustment hydrochloric acid or hydrobromic acid are preferred and especially preferred is hydrochloric acid.

The use of acidic solutions of acids which tend to form salts, especially hardly soluble or insoluble salts should be avoided. The acids which should be avoided are, for instance, sulfuric acid, phosphoric acid, acetic acid, citric acid, oxalic acid, malonic acid, salicylic acid, p-aminosalicylic acid, malic acid, fumaric acid, succinic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphorsulfonic acid, china acid, mandelic acid, o-methylmandelic acid, hydrogen-benzenesulfonic acid, picric acid, adipic acid, D-o-tolyltartaric acid, tartronic acid, (o, m, p)-toluic acid, naphthylamine sulfonic acid, trifluoroacetic acid.

Therefore, the present application is also directed to an ophthalmic formulation comprising or consisting of:

a) N-[5-(aminosulfonyl)-4-methyl-1,3-thiazol-2-yl]-N-methyl-2-[4-(2-pyridinyl)phenyl]acetamide hemihydrate,
b) polyethylene glycol, and
c) artificial tears solution,

wherein the ophthalmic formulation has a pH value in the range of from 5.0 to 7.0, preferably in the range of from 5.5 to 6.5.

Preferred is an ophthalmic formulation comprising or consisting of:

a) N-[5-(aminosulfonyl)-4-methyl-1,3-thiazol-2-yl]-N-methyl-2-[4-(2-pyridinyl)phenyl]acetamide hemihydrate,
b) PEG 200, PEG 300, PEG 400, or a mixture of two or more of these PEGs, and
c) artificial tears solution,

wherein the ophthalmic formulation has a pH value in the range of from 5.0 to 7.0, preferably in the range of from 5.5 to 6.5.

Also preferred is an ophthalmic formulation comprising or consisting of:

a) N-[5-(aminosulfonyl)-4-methyl-1,3-thiazol-2-yl]-N-methyl-2-[4-(2-pyridinyl)phenyl]acetamide hemihydrate,
b) PEG 200 and/or PEG 400, preferably PEG 400, and
c) artificial tears solution,

wherein the ophthalmic formulation has a pH value in the range of from 5.0 to 7.0, preferably in the range of from 5.5 to 6.5.

Also preferred is an ophthalmic formulation comprising or consisting of:

a) N-[5-(aminosulfonyl)-4-methyl-1,3-thiazol-2-yl]-N-methyl-2-[4-(2-pyridinyl)phenyl]acetamide hemihydrate,
b) polyethylene glycol, and
c) artificial tears solution,

wherein the ophthalmic formulation has a viscosity in the range of from 25 cp to 50 cp and a pH value in the range of from 5.0 to 7.0, preferably in the range of from 5.5 to 6.5.

**Artificial tears solution**

Artificial tears solutions also kown as tears replacement solutions are used to dilute or replace lacrimal fluid so that artificial tears solutions are used in ophthalmic formulations due to their eye compatibility.

Artificial tears solutions which could be used for the ophthalmic formulation of the present application are, for instance, Thera® Tears solution, GenTeal® Tears solution, Visine® Tears solution, Lac-Ophtal MP, Protagent®, Vidisept® EDO, WET-Comod®, Berberil® Dry Eye, Berberil® Dry Eye EDO, Artelac®, Yxin Teras®, Hylo-Comod®, Hyabak®, Artelac® splash MDO / EDO, Vislube®, Bepanthen eye drops (+ dexpanthenol), Opticalm eye drops (+ hypromellose), and others.

The artificial tears solution can be purchased as ready-to-use solution or can be prepared from commonly used and well available substances.

Common ingredients of an artificial tears solution are glycerin, hypromellose, and polyethylene glycol as lubricant and in addition normally 10 to all of the following substances are contained: carboxymethylcellulose, dextran, polysorbate, polyvinyl alcohol, povidone, propylene glycol, ascorbic acid, benzalkonium chloride, boric acid, dextrose, disodium phosphate, glycine, magnesium chloride, potassium chloride, sodium borate, sodium chloride, sodium citrate, and/or sodium lactate. All these substances are dissolved in purified water.

In accordance with the experiments performed and disclosed herein, the Visine® Tears solution or an equivalent artificial tears solution is the preferred artificial tears solution for the ophthalmic formulation.

The Visine® Tears solution contains 0.2% by weight glycerin, 0.2% by weight hypromellose, and 1% by weight PEG 400. In addition to these lubricants, the following substances are present in Visine® Tears solution: carboxymethylcellulose, dextran, polysorbate, polyvinyl alcohol, povidone, propylene glycol, ascorbic acid, benzalkonium chloride, boric acid, dextrose, disodium phosphate, glycine, magnesium chloride, potassium chloride, sodium borate, sodium chloride, sodium citrate, and sodium lactate. An artificial tears solution which is equivalent to the Visine® Tears solution contains substantially the same ingredients. However, it might lack one or more of the afore-mentioned ingredients or additionally contain one or more pharmaceutically accaptable ingredients as long as it exhibits subtantially the same properties of the Visine® Tears solution. An artificial tears solution which is equivalent to the Visine® Tears solution may also contain some or all of the afore-mentioned ingedients in different amounts as long as it exhibits subtantially the same properties of the Visine® Tears solution. The term "substantially the same properties" as used in the present application refers to the physicochemical characteristics of a particular formulation which is acceptable for use as an artificial tears solution. For example, an artificial tears solution which has substantially the same properties as the Visine® Tears solution has a pH which differs not more than 5% from the pH of a Visine® Tears solution and/or a viscosity which differs not more than 5% from the viscosity of a Visine® Tears solution and/or a density which differs not more than 10% of from the density of a Visine® Tears solution.

Therefore, the present application is also directed to an ophthalmic formulation comprising or consisting of:

a) N-[5-(aminosulfonyl)-4-methyl-1,3-thiazol-2-yl]-N-methyl-2-[4-(2-pyridinyl)phenyl]acetamide hemihydrate,
b) polyethylene glycol, and
c) artificial tears solution containing glycerin, hypromellose, and polyethylene glycol 400 and optionally povidone.

Also preferred is an ophthalmic formulation comprising or consisting of:

a) N-[5-(aminosulfonyl)-4-methyl-1,3-thiazol-2-yl]-N-methyl-2-[4-(2-pyridinyl)phenyl]acetamide hemihydrate,
b) PEG 200, PEG 300, PEG 400, or a mixture of two or more of these PEGs, and
c) artificial tears solution containing glycerin, hypromellose, and polyethylene glycol 400 and optionally povidone.

Also preferred is an ophthalmic formulation comprising or consisting of:

a) N-[5-(aminosulfonyl)-4-methyl-1,3-thiazol-2-yl]-N-methyl-2-[4-(2-pyridinyl)phenyl]acetamide hemihydrate,
b) PEG 200 and/or PEG 400, preferably PEG 400, and
c) artificial tears solution containing glycerin, hypromellose, and polyethylene glycol 400 and optionally povidone.

Also preferred is an ophthalmic formulation comprising or consisting of:

a) N-[5-(aminosulfonyl)-4-methyl-1,3-thiazol-2-yl]-N-methyl-2-[4-(2-pyridinyl)phenyl]acetamide hemihydrate,
b) polyethylene glycol, and
c) artificial tears solution containing glycerin, hypromellose, polyethylene glycol 400, carboxymethylcellulose, dextran, polysorbate, polyvinyl alcohol, povidone, propylene glycol, ascorbic acid, benzalkonium chloride, boric acid, dextrose, disodium phosphate, glycine, magnesium chloride, potassium chloride, sodium borate, sodium chloride, sodium citrate, and sodium lactate.

Also preferred is an ophthalmic formulation comprising or consisting of:

a) N-[5-(aminosulfonyl)-4-methyl-1,3-thiazol-2-yl]-N-methyl-2-[4-(2-pyridinyl)phenyl]acetamide hemihydrate,
b) PEG 200, PEG 300, PEG 400, or a mixture of two or more of these PEGs, and
c) artificial tears solution containing glycerin, hypromellose, polyethylene glycol 400, carboxymethylcellulose, dextran, polysorbate, polyvinyl alcohol, povidone, propylene glycol, ascorbic acid, benzalkonium chloride, boric acid, dextrose, disodium phosphate, glycine, magnesium chloride, potassium chloride, sodium borate, sodium chloride, sodium citrate, and sodium lactate.

Also preferred is an ophthalmic formulation comprising or consisting of:

a) N-[5-(aminosulfonyl)-4-methyl-1,3-thiazol-2-yl]-N-methyl-2-[4-(2-pyridinyl)phenyl]acetamide hemihydrate,
b) PEG 200 and/or PEG 400, preferably PEG 400, and
c) artificial tears solution containing glycerin, hypromellose, polyethylene glycol 400, carboxymethylcellulose, dextran, polysorbate, polyvinyl alcohol, povidone, propylene glycol, ascorbic acid, benzalkonium chloride, boric acid, dextrose, disodium phosphate, glycine, magnesium chloride, potassium chloride, sodium borate, sodium chloride, sodium citrate, and sodium lactate.

Also preferred is an ophthalmic formulation comprising or consisting of:

a) N-[5-(aminosulfonyl)-4-methyl-1,3-thiazol-2-yl]-N-methyl-2-[4-(2-pyridinyl)phenyl]acetamide hemihydrate,
b) polyethylene glycol, and
c) Visine® Tears solution.

Also preferred is an ophthalmic formulation comprising or consisting of:

a) N-[5-(aminosulfonyl)-4-methyl-1,3-thiazol-2-yl]-N-methyl-2-[4-(2-pyridinyl)phenyl]acetamide hemihydrate,
b) PEG 200, PEG 300, PEG 400, or a mixture of two or more of these PEGs, and
c) Visine® Tears solution.

Also preferred is an ophthalmic formulation comprising or consisting of:

a) N-[5-(aminosulfonyl)-4-methyl-1,3-thiazol-2-yl]-N-methyl-2-[4-(2-pyridinyl)phenyl]acetamide hemihydrate,
b) PEG 200 and/or PEG 400, preferably PEG 400, and
c) Visine® Tears solution.

Also preferred is an ophthalmic formulation comprising or consisting of:

a) N-[5-(aminosulfonyl)-4-methyl-1,3-thiazol-2-yl]-N-methyl-2-[4-(2-pyridinyl)phenyl]acetamide hemihydrate,
b) polyethylene glycol, and
c) Visine® Tears solution,

wherein the ophthalmic formulation has a pH value in the range of from 5.0 to 7.0, preferably in the range of from 5.5 to 6.5.
Also preferred is an ophthalmic formulation comprising or consisting of:

a) N-[5-(aminosulfonyl)-4-methyl-1,3-thiazol-2-yl]-N-methyl-2-[4-(2-pyridinyl)phenyl]acetamide hemihydrate,
b) PEG 200, PEG 300, PEG 400, or a mixture of two or more of these PEGs, and
c) Visine® Tears solution,

wherein the ophthalmic formulation has a pH value in the range of from 5.0 to 7.0, preferably in the range of from 5.5 to 6.5.
Also preferred is an ophthalmic formulation comprising or consisting of:

a) N-[5-(aminosulfonyl)-4-methyl-1,3-thiazol-2-yl]-N-methyl-2-[4-(2-pyridinyl)phenyl]acetamide hemihydrate,
b) PEG 200 and/or PEG 400, preferably PEG 400, and
c) Visine® Tears solution,

wherein the ophthalmic formulation has a pH value in the range of from 5.0 to 7.0, preferably in the range of from 5.5 to 6.5.
Concerning the present ophthalmic formulation it is preferred that the polyethylene glycol is contained in the

ophthalmic formulation in an amount of from 20 μg per ml ophthalmic formulation to 100 μg per ml ophthalmic formulation, more preferably in an amount of from 30 μg per ml ophthalmic formulation to 90 μg per ml ophthalmic formulation, more preferably in an amount of from 35 μg per ml ophthalmic formulation to 80 μg per ml ophthalmic formulation, more preferably in an amount of from 40 μg per ml ophthalmic formulation to 70 μg per ml ophthalmic formulation, still more preferably in an amount of from 45 μg per ml ophthalmic formulation to 65 μg per ml ophthalmic formulation, still more preferably in an amount of from 50 μg per ml ophthalmic formulation to 60 μg per ml ophthalmic formulation.

Therefore, the present application is also directed to an ophthalmic formulation comprising or consisting of:

a) N-[5-(aminosulfonyl)-4-methyl-1,3-thiazol-2-yl]-N-methyl-2-[4-(2-pyridinyl)phenyl]acetamide hemihydrate,
b) polyethylene glycol, and
c) artificial tears solution,

wherein the polyethylene glycol is contained in the ophthalmic formulation in an amount of from 40 μg per ml ophthalmic formulation to 70 μg per ml ophthalmic formulation.

Also preferred is an ophthalmic formulation comprising or consisting of:

a) N-[5-(aminosulfonyl)-4-methyl-1,3-thiazol-2-yl]-N-methyl-2-[4-(2-pyridinyl)phenyl]acetamide hemihydrate,
b) PEG 200, PEG 300, PEG 400, or a mixture of two or more of these PEGs, and
c) artificial tears solution,

wherein the polyethylene glycol is contained in the ophthalmic formulation in an amount of from 40 μg per ml ophthalmic formulation to 70 μg per ml ophthalmic formulation.

Also preferred is an ophthalmic formulation comprising or consisting of:

a) N-[5-(aminosulfonyl)-4-methyl-1,3-thiazol-2-yl]-N-methyl-2-[4-(2-pyridinyl)phenyl]acetamide hemihydrate,
b) PEG 200 and/or PEG 400, preferably PEG 400, and
c) artificial tears solution,

wherein the polyethylene glycol is contained in the ophthalmic formulation in an amount of from 40 μg per ml ophthalmic formulation to 70 μg per ml ophthalmic formulation.

Also preferred is an ophthalmic formulation comprising or consisting of:

a) N-[5-(aminosulfonyl)-4-methyl-1,3-thiazol-2-yl]-N-methyl-2-[4-(2-pyridinyl)phenyl]acetamide hemihydrate,
b) polyethylene glycol, and
c) Visine® Tears solution,

wherein the polyethylene glycol is contained in the ophthalmic formulation in an amount of from 40 μg per ml ophthalmic formulation to 70 μg per ml ophthalmic formulation.

Concerning the present ophthalmic formulation it is preferred that the N-[5-(aminosulfonyl)-4-methyl-1,3-thiazol-2-yl]-N-methyl-2-[4-(2-pyridinyl)-phenyl]acetamide hemihydrate is contained in the ophthalmic formulation in an amount of from 20 μg per ml ophthalmic formulation to 100 μg per ml ophthalmic formulation, more preferably in an amount of from 25 μg per ml ophthalmic formulation to 90 μg per ml ophthalmic formulation, more preferably in an amount of from 30 μg per ml ophthalmic formulation to 80 μg per ml ophthalmic formulation, more preferably in an amount of from 35 μg per ml ophthalmic formulation to 70 μg per ml ophthalmic formulation, still more preferably in an amount of from 40 μg per ml ophthalmic formulation to 60 μg per ml ophthalmic formulation, still more preferably in an amount of from 45 μg per ml ophthalmic formulation to 55 μg per ml ophthalmic formulation.

Therefore, the present application is also directed to an ophthalmic formulation comprising or consisting of:

a) N-[5-(aminosulfonyl)-4-methyl-1,3-thiazol-2-yl]-N-methyl-2-[4-(2-pyridinyl)phenyl]acetamide hemihydrate,
b) polyethylene glycol, and
c) artificial tears solution,

wherein the N-[5-(aminosulfonyl)-4-methyl-1,3-thiazol-2-yl]-N-methyl-2-[4-(2-pyridinyl)-phenyl]acetamide hemihydrate is contained in the ophthalmic formulation in an amount of from 40 μg per ml ophthalmic formulation to 60 μg per ml ophthalmic formulation.

Also preferred is an ophthalmic formulation comprising or consisting of:

a) N-[5-(aminosulfonyl)-4-methyl-1,3-thiazol-2-yl]-N-methyl-2-[4-(2-pyridinyl)phenyl]acetamide hemihydrate,

b) PEG 200, PEG 300, PEG 400, or a mixture of two or more of these PEGs, and
c) artificial tears solution,

wherein the N-[5-(aminosulfonyl)-4-methyl-1,3-thiazol-2-yl]-N-methyl-2-[4-(2-pyridinyl)-phenyl]acetamide hemihydrate is contained in the ophthalmic formulation in an amount of from 40 μg per ml ophthalmic formulation to 60 μg per ml ophthalmic formulation.

Also preferred is an ophthalmic formulation comprising or consisting of:

a) N-[5-(aminosulfonyl)-4-methyl-1,3-thiazol-2-yl]-N-methyl-2-[4-(2-pyridinyl)phenyl]acetamide hemihydrate,
b) PEG 200 and/or PEG 400, preferably PEG 400, and
c) artificial tears solution,

wherein the N-[5-(aminosulfonyl)-4-methyl-1,3-thiazol-2-yl]-N-methyl-2-[4-(2-pyridinyl)-phenyl]acetamide hemihydrate is contained in the ophthalmic formulation in an amount of from 40 μg per ml ophthalmic formulation to 60 μg per ml ophthalmic formulation.

Also preferred is an ophthalmic formulation comprising or consisting of:

a) N-[5-(aminosulfonyl)-4-methyl-1,3-thiazol-2-yl]-N-methyl-2-[4-(2-pyridinyl)phenyl]acetamide hemihydrate,
b) polyethylene glycol, and
c) Visine® Tears solution,

wherein the N-[5-(aminosulfonyl)-4-methyl-1,3-thiazol-2-yl]-N-methyl-2-[4-(2-pyridinyl)-phenyl]acetamide hemihydrate is contained in the ophthalmic formulation in an amount of from 40 μg per ml ophthalmic formulation to 60 μg per ml ophthalmic formulation.

Also preferred is an ophthalmic formulation comprising or consisting of:

a) N-[5-(aminosulfonyl)-4-methyl-1,3-thiazol-2-yl]-N-methyl-2-[4-(2-pyridinyl)phenyl]acetamide hemihydrate,
b) polyethylene glycol, and
c) artificial tears solution,

wherein the polyethylene glycol is contained in the ophthalmic formulation in an amount of from 40 μg per ml ophthalmic formulation to 70 μg per ml ophthalmic formulation and the N-[5-(aminosulfonyl)-4-methyl-1,3-thiazol-2-yl]-N-methyl-2-[4-(2-pyridinyl)-phenyl]acetamide hemihydrate is contained in the ophthalmic formulation in an amount of from 40 μg per ml ophthalmic formulation to 60 μg per ml ophthalmic formulation.

Also preferred is an ophthalmic formulation comprising or consisting of:

a) N-[5-(aminosulfonyl)-4-methyl-1,3-thiazol-2-yl]-N-methyl-2-[4-(2-pyridinyl)phenyl]acetamide hemihydrate,
b) PEG 200, PEG 300, PEG 400, or a mixture of two or more of these PEGs, and
c) artificial tears solution,

wherein the polyethylene glycol is contained in the ophthalmic formulation in an amount of from 40 μg per ml ophthalmic formulation to 70 μg per ml ophthalmic formulation and the N-[5-(aminosulfonyl)-4-methyl-1,3-thiazol-2-yl]-N-methyl-2-[4-(2-pyridinyl)-phenyl]acetamide hemihydrate is contained in the ophthalmic formulation in an amount of from 40 μg per ml ophthalmic formulation to 60 μg per ml ophthalmic formulation.

Also preferred is an ophthalmic formulation comprising or consisting of:

a) N-[5-(aminosulfonyl)-4-methyl-1,3-thiazol-2-yl]-N-methyl-2-[4-(2-pyridinyl)phenyl]acetamide hemihydrate,
b) PEG 200 and/or PEG 400, preferably PEG 400, and
c) artificial tears solution,

wherein the polyethylene glycol is contained in the ophthalmic formulation in an amount of from 40 μg per ml ophthalmic formulation to 70 μg per ml ophthalmic formulation and the N-[5-(aminosulfonyl)-4-methyl-1,3-thiazol-2-yl]-N-methyl-2-[4-(2-pyridinyl)-phenyl]acetamide hemihydrate is contained in the ophthalmic formulation in an amount of from 40 μg per ml ophthalmic formulation to 60 μg per ml ophthalmic formulation.

Also preferred is an ophthalmic formulation comprising or consisting of:

a) N-[5-(aminosulfonyl)-4-methyl-1,3-thiazol-2-yl]-N-methyl-2-[4-(2-pyridinyl)phenyl]acetamide hemihydrate,
b) polyethylene glycol, and

c) Visine® Tears solution,

wherein the polyethylene glycol is contained in the ophthalmic formulation in an amount of from 40 μg per ml ophthalmic formulation to 70 μg per ml ophthalmic formulation and the N-[5-(aminosulfonyl)-4-methyl-1,3-thiazol-2-yl]-N-methyl-2-[4-(2-pyridinyl)-phenyl]acetamide hemihydrate is contained in the ophthalmic formulation in an amount of from 40 μg per ml ophthalmic formulation to 60 μg per ml ophthalmic formulation.

Also preferred is an ophthalmic formulation comprising or consisting of:

a) N-[5-(aminosulfonyl)-4-methyl-1,3-thiazol-2-yl]-N-methyl-2-[4-(2-pyridinyl)phenyl]acetamide hemihydrate,
b) polyethylene glycol, and
c) artificial tears solution,

wherein the N-[5-(aminosulfonyl)-4-methyl-1,3-thiazol-2-yl]-N-methyl-2-[4-(2-pyridinyl)-phenyl]acetamide hemihydrate is contained in the ophthalmic formulation in an amount of from 40 μg per ml ophthalmic formulation to 60 μg per ml ophthalmic formulation and the ophthalmic formulation has a pH value in the range of from 5.0 to 7.0, preferably in the range of from 5.5 to 6.5.

Also preferred is an ophthalmic formulation comprising or consisting of:

a) N-[5-(aminosulfonyl)-4-methyl-1,3-thiazol-2-yl]-N-methyl-2-[4-(2-pyridinyl)phenyl]acetamide hemihydrate,
b) PEG 200, PEG 300, PEG 400, or a mixture of two or more of these PEGs, and
c) artificial tears solution,

wherein the N-[5-(aminosulfonyl)-4-methyl-1,3-thiazol-2-yl]-N-methyl-2-[4-(2-pyridinyl)-phenyl]acetamide hemihydrate is contained in the ophthalmic formulation in an amount of from 40 μg per ml ophthalmic formulation to 60 μg per ml ophthalmic formulation and the ophthalmic formulation has a pH value in the range of from 5.0 to 7.0, preferably in the range of from 5.5 to 6.5.

Also preferred is an ophthalmic formulation comprising or consisting of:

a) N-[5-(aminosulfonyl)-4-methyl-1,3-thiazol-2-yl]-N-methyl-2-[4-(2-pyridinyl)phenyl]acetamide hemihydrate,
b) PEG 200 and/or PEG 400, preferably PEG 400, and
c) artificial tears solution,

wherein the N-[5-(aminosulfonyl)-4-methyl-1,3-thiazol-2-yl]-N-methyl-2-[4-(2-pyridinyl)-phenyl]acetamide hemihydrate is contained in the ophthalmic formulation in an amount of from 40 μg per ml ophthalmic formulation to 60 μg per ml ophthalmic formulation and the ophthalmic formulation has a pH value in the range of from 5.0 to 7.0, preferably in the range of from 5.5 to 6.5.

Also preferred is an ophthalmic formulation comprising or consisting of:

a) N-[5-(aminosulfonyl)-4-methyl-1,3-thiazol-2-yl]-N-methyl-2-[4-(2-pyridinyl)phenyl]acetamide hemihydrate,
b) polyethylene glycol, and
c) Visine® Tears solution,

wherein the N-[5-(aminosulfonyl)-4-methyl-1,3-thiazol-2-yl]-N-methyl-2-[4-(2-pyridinyl)-phenyl]acetamide hemihydrate is contained in the ophthalmic formulation in an amount of from 40 μg per ml ophthalmic formulation to 60 μg per ml ophthalmic formulation and the ophthalmic formulation has a pH value in the range of from 5.0 to 7.0, preferably in the range of from 5.5 to 6.5.

Also preferred is an ophthalmic formulation comprising or consisting of:

a) N-[5-(aminosulfonyl)-4-methyl-1,3-thiazol-2-yl]-N-methyl-2-[4-(2-pyridinyl)phenyl]acetamide hemihydrate,
b) polyethylene glycol, and
c) artificial tears solution,

wherein the polyethylene glycol is contained in the ophthalmic formulation in an amount of from 40 μg per ml ophthalmic formulation to 70 μg per ml ophthalmic formulation and the N-[5-(aminosulfonyl)-4-methyl-1,3-thiazol-2-yl]-N-methyl-2-[4-(2-pyridinyl)-phenyl]acetamide hemihydrate is contained in the ophthalmic formulation in an amount of from 40 μg per ml ophthalmic formulation to 60 μg per ml ophthalmic formulation and the ophthalmic formulation has a pH value in the range of from 5.0 to 7.0, preferably in the range of from 5.5 to 6.5.

Also preferred is an ophthalmic formulation comprising or consisting of:

a) N-[5-(aminosulfonyl)-4-methyl-1,3-thiazol-2-yl]-N-methyl-2-[4-(2-pyridinyl)phenyl]acetamide hemihydrate,
b) PEG 200, PEG 300, PEG 400, or a mixture of two or more of these PEGs, and
c) artificial tears solution,

wherein the polyethylene glycol is contained in the ophthalmic formulation in an amount of from 40 μg per ml ophthalmic formulation to 70 μg per ml ophthalmic formulation and the N-[5-(aminosulfonyl)-4-methyl-1,3-thiazol-2-yl]-N-methyl-2-[4-(2-pyridinyl)-phenyl]acetamide hemihydrate is contained in the ophthalmic formulation in an amount of from 40 μg per ml ophthalmic formulation to 60 μg per ml ophthalmic formulation and the ophthalmic formulation has a pH value in the range of from 5.0 to 7.0, preferably in the range of from 5.5 to 6.5.

Also preferred is an ophthalmic formulation comprising or consisting of:

a) N-[5-(aminosulfonyl)-4-methyl-1,3-thiazol-2-yl]-N-methyl-2-[4-(2-pyridinyl)phenyl]acetamide hemihydrate,
b) PEG 200 and/or PEG 400, preferably PEG 400, and
c) artificial tears solution,

wherein the polyethylene glycol is contained in the ophthalmic formulation in an amount of from 40 μg per ml ophthalmic formulation to 70 μg per ml ophthalmic formulation and the N-[5-(aminosulfonyl)-4-methyl-1,3-thiazol-2-yl]-N-methyl-2-[4-(2-pyridinyl)-phenyl]acetamide hemihydrate is contained in the ophthalmic formulation in an amount of from 40 μg per ml ophthalmic formulation to 60 μg per ml ophthalmic formulation and the ophthalmic formulation has a pH value in the range of from 5.0 to 7.0, preferably in the range of from 5.5 to 6.5.

Also preferred is an ophthalmic formulation comprising or consisting of:

a) N-[5-(aminosulfonyl)-4-methyl-1,3-thiazol-2-yl]-N-methyl-2-[4-(2-pyridinyl)phenyl]acetamide hemihydrate,
b) polyethylene glycol, and
c) Visine® Tears solution,

wherein the polyethylene glycol is contained in the ophthalmic formulation in an amount of from 40 μg per ml ophthalmic formulation to 70 μg per ml ophthalmic formulation and the N-[5-(aminosulfonyl)-4-methyl-1,3-thiazol-2-yl]-N-methyl-2-[4-(2-pyridinyl)-phenyl]acetamide hemihydrate is contained in the ophthalmic formulation in an amount of from 40 μg per ml ophthalmic formulation to 60 μg per ml ophthalmic formulation and the ophthalmic formulation has a pH value in the range of from 5.0 to 7.0, preferably in the range of from 5.5 to 6.5.

Concerning the present ophthalmic formulation it is preferred that the artificial tears solution is contained in the ophthalmic formulation in an amount of from 0.5 g per ml ophthalmic formulation to 1.7 g per ml ophthalmic formulation, more preferably in an amount of from 0.6 g per ml ophthalmic formulation to 1.6 g per ml ophthalmic formulation, more preferably in an amount of from 0.7 g per ml ophthalmic formulation to 1.5 g per ml ophthalmic formulation, still more preferably in an amount of from 0.8 g per ml ophthalmic formulation to 1.4 g per ml ophthalmic formulation, still more preferably in an amount of from 0.9 g per ml ophthalmic formulation to 1.3 g per ml ophthalmic formulation, still more preferably in an amount of from 1.0 g per ml ophthalmic formulation to 1.2 g per ml ophthalmic formulation.

The ophthalmic formulation of the present application is used for prophylaxis and/or treatment of diseases, caused by herpes simplex viruses, and/or prevention of transmission of a herpes virus or herpes viruses.

Preferably, the ophthalmic formulation of the present application is used for prophylaxis and/or treatment of herpes simplex infections of the eye, especially of ocular herpes (i.e. keratitis).

Thus, in some preferred embodiments, the present application is directed to an ophthalimic formulation comprising:

a) N-[5-(aminosulfonyl)-4-methyl-1,3-thiazol-2-yl]-N-methyl-2-[4-(2-pyridinyl)phenyl]acetamide hemihydrate,
b) polyethylene glycol, and
c) artificial tears solution,

for use in the prophylaxis and/or treatment of diseases caused by herpes simplex viruses, and/or prevention of transmission of a herpes virus or herpes viruses. The diseases caused by herpes simplex viruses are preferably herpes simplex infections of the eye, especially of ocular herpes (i.e. keratitis).

Consequently, an aspect of the present application is the use of N-[5-(aminosulfonyl)-4-methyl-1,3-thiazol-2-yl]-N-methyl-2-[4-(2-pyridinyl)phenyl]acetamide hemihydrate in the prophylaxis and/or treatment of diseases caused by herpes simplex viruses, and/or in the prevention of transmission of a herpes virus or herpes viruses. The N-[5-(aminosulfonyl)-4-methyl-1,3-thiazol-2-yl]-N-methyl-2-[4-(2-pyridinyl)phenyl]acetamide hemihydrate is preferably administered as ophthalimic formulation as disclosed herein. Preferred is an administration of one to five drops of an ophthalimic formulation as disclosed herein two or three times a day.

As used herein the term "prevent", "preventing", "prevention" or "prophylaxis" means no disorder or disease development if none had occurred, or no further disorder or disease development if there had already been development of the disorder or disease. Also considered is the ability of one to prevent some or all of the symptoms associated with the disorder or disease. Prevention of diseases means prophylaxis of diseases and prophylaxis of diseases means prevention of diseases. Thus, the terms "prophylaxis" and "prevention" are used interchangeably throughout the present application.

The ophthalmic formulation of the present application can also be used together or in combination with a further antiviral agent such as an antimetabolite and preferably a nucleobase analogue, nucleotide analogues or nucleoside analogue drug. It is further preferred if the further antiviral agent is useful against herpes viruses and/or against transmission of a herpes virus or herpes viruses and is selected from the group of drugs comprising but not limited to or consisting of: acetylsalicylic acid, trifluridine, idoxuridine, foscarnet, cidofovir, ganciclovir, acyclovir, penciclovir, or the respective prodrugs valaciclovir, valganciclovir, or famciclovir.

Another aspect of the present invention is directed to a method of prophylaxis or treatment of diseases caused by herpes simplex viruses, and/or prevention of transmission of a herpes virus or herpes viruses comprising: administering an effective amount of the ophthalmic formulation of the present invention comprising

a) N-[5-(aminosulfonyl)-4-methyl-1,3-thiazol-2-yl]-N-methyl-2-[4-(2-pyridinyl)phenyl]acetamide hemihydrate,
b) polyethylene glycol, and
c) artificial tears solution,

to a patient in need of such treatment. Preferably the diseases caused by herpes simplex viruse are herpes simplex infections of the eye, especially of ocular herpes (i.e. keratitis).

Said method can also be used together or in combination with a further antiviral agent such as an antimetabolite and preferably a nucleobase analogue, nucleotide analogues or nucleoside analogue drug. Preferably the further antiviral agent is effective against herpes viruses and/or against transmission of a herpes virus or herpes viruses and is preferably selected from the group of drugs comprising but not limited to or consisting of: acetylsalicylic acid, trifluridine, idoxuridine, foscarnet, cidofovir, ganciclovir, acyclovir, penciclovir, or the respective prodrugs valaciclovir, valganciclovir, or famciclovir.

Thus, an aspect of the present application is a method of prophylaxis or treatment of diseases caused by herpes simplex viruses, and/or prevention of transmission of a herpes virus or herpes viruses comprising administering an effective amount of N-[5-(aminosulfonyl)-4-methyl-1,3-thiazol-2-yl]-N-methyl-2-[4-(2-pyridinyl)phenyl]acetamide hemihydrate to a patient in need thereof. The N-[5-(aminosulfonyl)-4-methyl-1,3-thiazol-2-yl]-N-methyl-2-[4-(2-pyridinyl)phenyl]acetamide hemihydrate is preferably administered as ophthalimic formulation as disclosed herein. Preferred is an administration of one to five drops of an ophthalimic formulation as disclosed herein two or three times a day.

The N-[5-(aminosulfonyl)-4-methyl-1,3-thiazol-2-yl]-N-methyl-2-[4-(2-pyridinyl)phenyl]acetamide hemihydrate can also be administered together with a further antiviral agent such as an antimetabolite and preferably a nucleobase analogue, nucleotide analogues or nucleoside analogue drug. Preferred drugs are acetylsalicylic acid, trifluridine, idoxuridine, foscarnet, cidofovir, ganciclovir, acyclovir, penciclovir, valaciclovir, valganciclovir, or famciclovir. This further drug can be contained in the same ophthalimic formulation or in a further ophthalimic formulation which is administered at the same time or within certain time intervals in relation to administration of the N-[5-(aminosulfonyl)-4-methyl-1,3-thiazol-2-yl]-N-methyl-2-[4-(2-pyridinyl)phenyl]acetamide hemihydrate.

Another aspect of the present invention is directed to the use of N-[5-(aminosulfonyl)-4-methyl-1,3-thiazol-2-yl]-N-methyl-2-[4-(2-pyridinyl)phenyl]acetamide hemihydrate for the preaparation of an ophthalmic formulation comprising:

a) N-[5-(aminosulfonyl)-4-methyl-1,3-thiazol-2-yl]-N-methyl-2-[4-(2-pyridinyl)phenyl]acetamide hemihydrate,
b) polyethylene glycol, and
c) artificial tears solution,

for the prophylaxis and/or treatment of diseases caused by herpes simplex viruses, and/or prevention of transmission of a herpes virus or herpes viruses. Preferably such diseases caused by herpes simplex viruses are herpes simplex infections of the eye, especially of ocular herpes (i.e. keratitis).

A further aspect of the present application relates to a method for the preparation of the ophthalmic formulation, wherein the method comprises the following steps:

I) providing N-[5-(aminosulfonyl)-4-methyl-1,3-thiazol-2-yl]-N-methyl-2-[4-(2-pyridinyl)phenyl]acetamide hemihydrate,
II) providing polyethylene glycol,

III) providing artificial tears solution,

IV) preparing a stock solution of N-[5-(aminosulfonyl)-4-methyl-1,3-thiazol-2-yl]-N-methyl-2-[4-(2-pyridinyl)phenyl] acetamide hemihydrate in polyethylene glycol,

V) diluting the stock solution with artificial tears solution until the desired concentration is reached,

VI) adjusting the pH value by addition of an aqueous solution of an acid to the desired pH value, and

VII) performing a sterile filtration by means of a suitable sterile filter to obtain the ophthalmic formulation.

This method preferably comprises at least one step of adjusting the pH value to the desired pH value which is preferably in the range of from 5.5 to 6.5. The ophthalmic solution can be adjusted to the desired pH value before and after sterile filtration. However, all pH ranges disclosed herein would likewise be acceptable for preparing the ophthalmic formulation.

The ophthalmic formulation should be stored at a temperature in the range of from 2°C to 8°C, but can without degradation also be stored at room temperature.

The N-[5-(aminosulfonyl)-4-methyl-1,3-thiazol-2-yl]-N-methyl-2-[4-(2-pyridinyl)phenyl] acetamide hemihydrate is used within this method in the amounts and concentrations as disclosed above.

The same is true for the other ingredients such as the polyethylene glycol and the artificial tears solution which are both used in an amount and concentration as disclosed above. In the method described herein the use of PEG 400 and Visine® Tears solution as artificial tears solution is preferred.

The formulation is sterilized by filtration through a sterile / aseptic filter to produce a formulation that is sterile. The sterile filtration may be performed at any convenient temperature such as room temperature. The sterile filtration is through a filter with a pore size of preferably 0.20 μm or less.

The steril filter material includes, but not limited to, polyvinylidene fluoride (PVDF), polypropylene (PE), polyphenylene oxide, polytetrafluorethylene (PTFE), ethylene-tetrafluoroethylene copolymer, polysulfone, polyethersulphone (PES), polyacetate (PA)/Nylon, PA-LE/Nylon, polyethersulfone, celullose acetate (CA) and regenerated cellulose (RC).

However, it was found that the step of the sterile filtration is crucial and could only be performed using a particular type of sterile filter, namely a RC filter.

Regenerated cellulose (RC) is a class of materials manufactured by the conversion of natural cellulose to a soluble cellulosic derivative and subsequent regeneration, typically forming either a fiber (via polymer spinning) or a film (via polymer casting). The RC sterile filter is a hydrophilic membrane filter. Said RC sterile filter has a thickness in a range of 10 to 500 μm, preferably 50 to 400 μm, more preferably 100 to 300 μm, most preferably 150 to 200 μm. The RC sterile filter has a flow rate more than 10 mL/min*$cm^2$, preferably more than 15 mLlmin*$cm^2$ at 25°C at 0.7 bar.

The RC sterile filter was purchased from CZT (Klaus Trott Chromatographie-Zubehör, Kriftel, Germany; Article No. 802527020).

The RC sterile filter has a pore size of 0.2 μm and was connected via Luer Lok. The specication of said RC sterile filter is summarized below:

| CZT -802527020 | Specification | Result |
|---|---|---|
| Luer-lock | Enhanced Luer-Lock (ISO 594-1) | Pass |
| Bubble point | >250 kPa | 362 |
| Ext. dimensions | 31.9 ± 0.3 mm x 25.7 ± 0.3 mm (height) | |
| Water Flow Rate | >10 ml/min. $cm^2$ (at 25°C 0.7 bar) | 19.2 |
| UV extractable | According conditions specified below. Absorbance at | Water |
| | 214< 0.04 AU | 0.0170 |
| | 254 < 0.04 AU | 0.0040 |
| | 280 < 0.04 AU | 0.0020 |

Consequently, the present application relates to a method for the preparation of the ophthalmic formulation, wherein the method comprises the following steps:

I) providing N-[5-(aminosulfonyl)-4-methyl-1,3-thiazol-2-yl]-N-methyl-2-[4-(2-pyridinyl)phenyl]acetamide hemihydrate,

II) providing polyethylene glycol,

III) providing artificial tears solution,

IV) preparing a stock solution of N-[5-(aminosulfonyl)-4-methyl-1,3-thiazol-2-yl]-N-methyl-2-[4-(2-pyridinyl)phenyl] acetamide hemihydrate in polyethylene glycol,
V) diluting the stock solution with artificial tears solution until the desired concentration is reached,
VI) adjusting the pH value by addition of an aqueous solution of an acid to the desired pH value, and
VII) performing a sterial filtration by means of a RC sterile filter to obtain the ophthalmic formulation.

## EXAMPLES

**1. Abbreviation list**

| | |
|---|---|
| PA | Polyacetate |
| LE | Low extractable |
| PFTE | Polytetrafluorethylene |
| PVDF | Polyvinylidenfluoride |
| CM | Celullose acetate |
| RC | Regenerated cellulose |
| PP | Polypropylene |
| PES | Polyethersulphone |

2. **Materials**

| Description | Manufacturer | Art No. or PZN-No |
|---|---|---|
| 50.0mL tubes | Greiner-bio-one | 227261 |
| Sterile 2.0mL tubes | Eppendorf | 0030 120.094 |
| Sterile 5.0mL tubes | Eppendorf | 0030 119.401 |
| 150mL sterile Filter system 0.22μm | Corning | Ref 431154 |
| 10ml-syringe Plastipak Luer-Lock | VWR | SS 10LE1 |
| Syringe filter 25mm (PA / Nylon) 0.2μm | CZT | 802510020 |
| Syringe filter 25mm (PA / Nylon) 0.45μm | CZT | 802510045 |
| Syringe filter 25mm (PA-LE / Nylon) 0.2μm | CZT | 802512020 |
| Syringe filter 25mm (PA-LE / Nylon) 0.45μm | CZT | 802512045 |
| Syringe filter 25mm (PTFE) 0.2μm | CZT | 802520020 |
| Syringe filter 25mm (PTFE) 0.45μm | CZT | 802520045 |
| Syringe filter 25mm (PVDF) 0.2μm | CZT | 802530020 |
| Syringe filter 25mm (PVDF) 0.45μm | CZT | 802530045 |
| Syringe filter 25mm (CM) 0.2μm | CZT | 802526020 |
| Syringe filter 25mm (CM) 0.45μm | CZT | 802526045 |
| Syringe filter 25mm (CA) 0.2μm | CZT | 802525020 |
| Syringe filter 25mm (CA) 0.45μm | CZT | 802525045 |
| Syringe filter 25mm (RC) 0.2μm | CZT | 802527020 |
| Syringe filter 25mm (RC) 0.45μm | CZT | 802527045 |

(continued)

| Description | Manufacturer | Art No. or PZN-No |
|---|---|---|
| Syringe filter 25mm (PP) 0.2μm | CZT | 802516020 |
| Syringe filter 25mm (PP) 0.45μm | CZT | 802516045 |
| Syringe filter 25mm (PES) 0.2μm | CZT | 802540020 |
| Syringe filter 25mm (PES) 0.45μm | CZT | 802540045 |

**3. Equipment**

| Equipment | Supplier |
|---|---|
| Climate chamber | Weißtechnik |
| pH meter 766 Calimatic | Knick |
| electrode SE104N | Knick |

**4. Analytical Equipment**

| Equipment | Name (Identifier) | Supplier |
|---|---|---|
| Autosampler | HTS PAL | CTC Analytics |
| Binary pump Column oven Degasser Diode array detector (DAD) | 1200 Series | Agilent Technologies |

**5. Preparation protocol HPLC Analysis**

**5.1 Quantification**

**5.1.1 Preparation of the Eluents**

- Add 10mL HAc to 990mL water and mix it
- Add 495mL Methanol to 495mL ACN and 10mL HAc and mix it

**5.2 Sample preparation**

**Ophthalmic formulation con = 0.05mg AIC090093 /mL**

**Related impurities**

- Dilute 500μL of each sample with 500μL ACN/MeOH/$H_2O$ ratio 25:25:50 + 1% HAc (1:2)
- Mix it 5s by vortexing

**Final concentration**

- Dilute 250μL of each sample with 730μL ACN/MeOH/$H_2O$ ratio 25:25:50 + 1% HAc and add 20μL ACN/DMSO 4:1 (1:4)
- Mix it 5s by vortexing

### 5.3 Calibration stock solution 10mg/ml AIC090093

• Weigh AIC090093 and solve in 10mL DMSO
• Mix it 15min in the Vortex

| | Target weight |
|---|---|
| AIC090093 | 100.0mg |
| DMSO | 10mL |

### 5.4 Vehicle solution

• Mix PEG 200 and GenTeal Tears
• Mix it 15min in the Vortex

| | Target volume |
|---|---|
| PEG | 0.75mL |
| Visine Tears | 14.25mL |

### 5.5 Calibration, Qualification and Assay working standard solution prepration

| | Concentration AIC090093 [mg/mL] | Volume diluent ACN/ DMSO 4+1 [μl] | Calibration stock solution [μl] |
|---|---|---|---|
| Blank | 0 | 1000 | 0 |
| Kal1 | 0.120 | 988 | 12 |
| Kal2 | 0.250 | 975 | 25 |
| Kal3 | 0.500 | 950 | 50 |
| Kal4 | 0.750 | 925 | 75 |
| Kal5 | 1.000 | 900 | 100 |
| Kal6 | 2.000 | 850 | 200 |
| Kal7 | 2.500 | 750 | 250 |
| Kal8 | 3.500 | 650 | 350 |
| QAL1 | 0.280 | 872 | 28 |
| QAL2 | 1.500 | 850 | 150 |
| QAL3 | 3.000 | 700 | 300 |
| AWS | 0.200 | 980 | 20 |

### 5.6 Dilution for HPLC

• • Dilute 20μl calibration or QC-solution with 730μl ACN/MeOH/$H_2O$ ratio 25:25:50 + 1% HAc and add 250μL of the corresponding vehicle solution (1:50)

| | Concentration [mg/ml] |
|---|---|
| Blank | 0 |
| K1 | 0.002 |

(continued)

| | Concentration [mg/ml] |
|---|---|
| K2 | 0.005 |
| K3 | 0.010 |
| K4 | 0.015 |
| K5 | 0.020 |
| K6 | 0.040 |
| K7 | 0.050 |
| K8 | 0.075 |
| QC1 | 0.0056 |
| QC2 | 0.030 |
| QC3 | 0.060 |
| AWS | 0.0040 |

**5.7 Measuring HPLC-DAD**

| | |
|---|---|
| Method: | AIC090093_V1 |
| Column: | Luna C18(2) 5μm, 150 x 2mm, Phenomenex #84 |
| Column oven temperature | 23°C |
| Auto Sampler-temperature. (CTC PAL) | 5°C |

**PAL Sampler**

| | |
|---|---|
| Injection Volume: | 10 μl |
| Overlap Injection Mode: | No Overlap |
| Module Display Name: | PAL |
| Module Type: | PAL Sampler |
| Order: | 1 |

**PAL Method Information**

Parameters of PAL Cycle

| | |
|---|---|
| Air Volume (μl): | 0 |
| Pre Clean with Solvent 1 (): | 0 |
| Pre Clean with Solvent 2 (): | 0 |
| Pre Clean with Sample (): | 0 |
| Filling Speed (μl/s): | 10 |
| Filling Strokes (): | 1 |
| Inject to: | LC Vlv1 |
| Injection Speed (μl/s): | 5 |
| Pre Inject Delay (ms): | 500 |
| Post Inject Delay (ms): | 500 |
| Post Clean with Solvent 1 (): | 2 |

(continued)

| | |
|---|---|
| Parameters of PAL Cycle | |
| Post Clean with Solvent 2 (): | 2 |
| Valve Clean with Solvent 1 (): | 1 |
| Valve Clean with Solvent 2 (): | 1 |

**DAD Method**

| | |
|---|---|
| Peakwidth: | > 0.1 min (2 s response time) (2.5 Hz) |
| Slit: | 4 nm |
| UV Lamp Required: | Yes |
| Vis Lamp Required: | Yes |
| Module Display Name: | DAD |
| Module Type: | G1315C |
| Order: | 1 |

**Analog Output 1**

| | |
|---|---|
| Analog 1 Zero Offset: | 5 % |
| Analog 1 Attenuation: | 1000 mAU |

**Analog Output 2**

| | |
|---|---|
| Analog 2 Zero Offset: | 5 % |
| Analog 2 Attenuation: | 1000 mAU |

**Signals**

Signal table

| Row ID | Acquire | Signal | Wavelength (nm) | Bandwidth (nm) |
|---|---|---|---|---|
| 1 | No | Signal A | | |
| 2 | Yes | Signal B | 290 | 20 |
| 3 | No | Signal C | | |
| 4 | No | Signal D | | |
| 5 | No | Signal E | | |
| 6 | No | Signal F | | |
| 7 | No | Signal G | | |
| 8 | No | Signal H | | |

| Row ID | Use Ref. | Ref Wavel. (nm) | Ref Bandw. (nm) |
|---|---|---|---|
| 1 | | | |
| 2 | Yes | 450 | 50 |
| 3 | | | |
| 4 | | | |
| 5 | | | |
| 6 | | | |
| 7 | | | |
| 8 | | | |

**Prepare Mode**

Margin for negative 100 mAU
Absorbance:

**Autobalance**

Autobalance Prerun: Yes
Autobalance Postrun: Yes

**Spectrum**

Spectrum Store: None

**Stoptime**

Stoptime Mode: As pump/injector

**Posttime**

Posttime Mode: Off

**Column Comp. Method**

Module Display Name: Column Comp
Module Type: G1316B
Order: 1

**Left Temperature Control**

| | |
|---|---|
| Temperature Control Mode: | Temperature Set |
| Temperature: | 23.0 °C |

**Enable Analysis Left Temperature**

| | | |
|---|---|---|
| Enable Analysis Temperature On: | Left | Yes |
| Enable Analysis Temperature Value: | Left | 1.0 °C |

**Right Temperature Control**

| | |
|---|---|
| Right temperature Control Mode: | Combined |

**Enable Analysis Right Temperature**

| | |
|---|---|
| Enable Analysis Right Temperature On: | Yes |
| Enable Analysis Right Temperature Value: | 1.0 °C |

**Stop Time**

| | |
|---|---|
| Stoptime Mode: | As pump/injector |

**Post Time**

| | |
|---|---|
| Posttime Mode: | Off |

**Binary Pump Method**

| | |
|---|---|
| Flow: | 0.350 mL/min |
| Use Solvent Types: | Yes |
| Low Pressure Limit: | 0.00 bar |
| High Pressure Limit: | 400.00 bar |
| Maximum Flow Gradient: | 100.000 mL/min$^2$ |
| Module Display Name: | Binary Pump |
| Module Type: | G1312B |
| Order: | 1 |

**Solvent Composition**

| Row ID | Channel | Solvent 1 | Name 1 | Used |
|---|---|---|---|---|
| 1 | A | $H_2O$ | 1% HAc | Yes |
| 2 | B | MeOH | ACN MeOH 1:1 1% HAc | Yes |

| Row ID | Percent (%) |
|---|---|
| 1 | 98.0 |
| 2 | 2.0 |

**Timetable**

| Row ID | Time (min) | A (%) | B (%) | Flow (mL/min) |
|---|---|---|---|---|
| 1 | 2.00 | 98.0 | 2.0 | 0.350 |
| 2 | 20.00 | 30.0 | 70.0 | 0.350 |
| 3 | 20.10 | 2.0 | 98.0 | 0.350 |
| 4 | 25.00 | 98.0 | 2.0 | 0.350 |

| Row ID | Pressure (bar) | Contact A | Contact B | Contact C |
|---|---|---|---|---|
| 1 | 400.00 | open | open | open |
| 2 | 400.00 | open | open | open |
| 3 | 400.00 | open | open | open |
| 4 | 400.00 | open | open | open |

| Row ID | Contact D |
|---|---|
| 1 | open |
| 2 | open |
| 3 | open |
| 4 | open |

**Stroke A**

| | |
|---|---|
| Automatic Calculation A: | Stroke Yes |

**Stroke B**

| | |
|---|---|
| Automatic Calculation B: | Stroke Yes |

**Stop Time**

| | |
|---|---|
| Stoptime Mode: | Time set |
| Stoptime: | 25.00 min |

**Post Time**

| | |
|---|---|
| Posttime Mode: | Off |

**External Contacts**

| | |
|---|---|
| Contact A: | Open |
| Contact B: | Open |
| Contact C: | Open |
| Contact D: | Open |

**Method Properties**

| | |
|---|---|
| Instrument Technique: | Liquid Chromatography |

**6. Preparation of the ophthalmic formulation**

Chemicals and materials:

Pritelivir free base hemihydrate was synthesized by Carbogen amics.
Pritelivir maleate was synthesized by Carbogen amics.
Pritelivir mesylate monohydrate was synthesized by Carbogen amics.
Polyethylenglycol 200 (Polygylcol 200) was obtained from Clariant.
Polyethylenglycol 400 (Kollisolv 400) was purchased from BASF.
GenTeal® Tears solution was obtained from Alcon.
Thera® Tears solution was obtained from Akron Consumer Health.
Visine® Tears solution was obtained from Johnson & Johnson Healthcare.

**Preparation Experiments**

The following experiments describe the preparation of several different ophthalmic formulations with different drug substances (DS). The term Pritelivir hemihydrate as used herein refers to Pritelivir free base hemihydrate and to N-[5-(aminosulfonyl)-4-methyl-1,3-thiazol-2-yl]-N-methyl-2-[4-(2-pyridinyl)phenyl]acetamide hemihydrate. The term Pritelivir maleate as used herein refers to to N-[5-(aminosulfonyl)-4-methyl-1,3-thiazol-2-yl]-N-methyl-2-[4-(2-pyridinyl)phenyl]acetamide maleate and the term Pritelivir mesylate monohydrate as used herein refers to N-[5-(amino-sulfonyl)-4-methyl-1,3-thiazol-2-yl]-N-methyl-2-[4-(2-pyridinyl)phenyl] acetamide mesylate monohydrate.

**Example 1**

In order to prepare the new ophthalmic formulation the drug substance concentrations of both i) stock solution and ii) drug product which is administered to the patient need to be determined. For that prupose, a stock solution with different concentrations of Pritelivir free base hemihydrate in PEG 200 was diluted in GenTeal® tears and the precipitation was checked over time.

| Experiment | DS | Excipients | Scope |
|---|---|---|---|
| 1 | Pritelivir hemihydrate | • PEG200<br>• GenTeal ® Tears | Preparation of stock solution with different concentrations of DS. Dilution in GenTeal® Tears with different final concentrations of the ophthalmic formulation. Check for precipitation over time in a pre-test. |

**Preparation of stock solutions**

All liquids and the DS were equilibrated to ≥ 20°C. Seven 10 mL tubes were provided. In each of these tubes, 5 ml of PEG 200 were added and the required amount of DS was dissolved therein to provide the following stock solutions: 30mg/mL, 25mg/mL, 20mg/mL, 10 mg/mL, 5 mg/mL, 1 mg/mL, 0.05 mg/mL. The solutions were mixed by vortexing the tubes for 5s and the tubes were shaken until the DS was dissolved.

**Preparation ophthalmic formulations**

The stock solutions were diluted with GenTeal® tears and the pH value was measured. After that the samples were checked for precipitation by visual inspection.
An ophthalmic formulation with a drug load of 24 mg/mL was obtainable without any visually detectable precipitation.

**Example 2**

To study the in-use stability over 5 weeks at room temperature (RT) and at a temperature in the range of from 2°C - 8°C, stock solutions with a concentration of 1.0 mg/mL of DS were prepared in PEG200 and in PEG400. The different stock solutions were diluted in 3 different compositions of artificial tears to a final concentration of 0.05 mg DS per mL ophthalmic formulation.

| Experiment | DS | Excipients | Scope |
|---|---|---|---|
| 2 | Pritelivir hemihydrate | • PEG200<br>• PEG400<br><br>• GenTeal® Tears<br>• Thera® Tears<br>• Visine® Tears | A stock solution in PEG with a concentration 1.0mg/mL of DS was prepared by using two different grades of PEG (PEG200 and PEG400).<br>The different stock solutions were diluted in 3 different brands of artificial tears (0.05mg/mL DS). |

**Preparation of stock solution 1mg/mL**

All liquids and the DS were equilibrated to ≥ 20°C. Amounts of 10 ml of the respective PEG (either PEG 200 or PEG 400) were added to a 50 mL tube and 10 mg of DS were dissolved in each tube. The solutions were mixed by vortexing the tubes for 5s and the tubes were shaken until the DS was dissolved.

**Preparation ophthalmic formulations 0.05mg/mL**

An amount of 1.5mL stock solution was diluted with 28.5mL of the respective artificial tears solution (either GenTeal® Tears or Thera® Tears or Visine® tears) in a beaker and stirred for 2min with a magnet stirrer. The pH value was determined and samples were taken before and after sterile filtration. The solutions were filtered through CA (cellulose acetate) 0.22 μm filter system. The content of the beaker was withdrawn and respectively 14mL of the prepared formulation were added to two empty 50mL tubes. In addition, one empty 2 mL tube was provided and 1.5mL of the prepared formulation was added to the 2mL tube. The samples were stored at RT and at a temperature in the range of from 2-8°C

over 5 weeks.

It was observed that the pH value of the ophthalmic formulation depended on the artificial tears solution used. The pH value of the artificial tears solution varied between 7.5 and 8.5.

However, it was quite surprising to note that after the sterile filtration of the ophthalmic formulation no DS could be detected by HPLC for all prepared formulations anymore.

**Example 3**

To study the filter compatibility a stock solution in PEG200 at a concentration 1.0mg Pritelivir free base hemihydrate per mL of stock solution was diluted in GenTeal® tears to a concentration of 0.05mg Pritelivir free base hemihydrate per ophthalmic formulation.

The ophthalmic formulation was filtered with different filter materials and pore sizes.

| Experiment | DS | Excipients | Scope |
|---|---|---|---|
| 3 | Pritelivir hemihydrate | • PEG200<br><br>• GenTeal® Tears | A stock solution in PEG200 with a concentration 1.0mg/mL of DS was diluted in GenTeal® tears (0.05mg/mL DS).<br>The final solution was filtered with different filter materials and pore sizes. |

**Preparation of stock solution 1mg/mL DS**

All liquids and the DS were equilibrated to ≥ 20°C. An amount of 10 ml of PEG 200 was added to a 50 mL tube and 10 mg of DS were dissolved therein. The solution was mixed by vortexing the tube for 5s and the tube was shaken until the DS was dissolved.

**Preparation of an ophthalmic formulation 0.05mg/mL DS**

The amount of 4.0mL stock solution was diluted with 76.0mL of GenTeal® tears and the solution was mixed by vortexing for 5s stirring for 2 min until the DS was dissolved.

**Filter testing for all syringe filters** (e.g. PA 0.2μm)

The testing was repeated one time to have samples in duplicate (n=2). An amount of 6 mL of the ophthalmic formulation was withdrawn with a 10mL syringe. An amount of 1mL was used to determine the final concentration before filtration. The syringe was connected with a PA 0.2μm filter via Luer Lok and the solution was filtered. Another sample was taken to determine the final concentration after filtration.

The filter was then disconnected from the syringe and an amount of 3mL DMSO was withdrawn with the used 10mL syringe. Then, the syringe was shaken smoothly. An amount of 1mL was used to determine the final concentration after rinsing. The syringe was again connected to the used PA 0.2μm filter via Luer Lok the DMSO was filtered. A sample was taken to determine the final concentration after washing.

This protocol was repeated for all tested filters: PA / Nylon, PALE / Nylon, PTFE, PVDF, CM, CA, RC, PP, PES. Each filter contained a filter material with a pore size of 0.2μm and of 0.45μm.

Surprisingly, only two filters were identified to be suitable for a sterile filtration of the ophthalmic formulation. The assay data before and after filtration of the filter materials PFTE 0.2μm and RC 0.2μm showed identical values.

**Example 4**

To study the feasibility of the sterile filtration with two grades of PEG and 3 different compositions of artificial tears, the experiment of Example 3 was repeated and, in addition the same conditions of the experiment of Example 3 were tested using a stock solution in PEG400 at a concentration 1.0mg Pritelivir free base hemihydrate per mL stock solution and 2 other compositions of commercial available artificial tears.

| Experiment | DS | Excipients | Scope |
|---|---|---|---|
| 4 | Pritelivir hemihydrate | • PEG200<br>• PEG400<br>• GenTeal® Tears<br>• Thera® Tears<br>• Visine® Tears | The experiments 3 were repeated for confirmation. In addition the tests were repeated by using a stock solution in PEG400 with a concentration 1.0mg/mL of DS and 2 other brands of commercially available artificial tears solutions. |

**Preparation of PEG200 stock solution 1mg/mL DS**

All liquids and the DS were equilibrated to ≥ 20°C. An amount of 10 ml of PEG 200 was added to a 50 mL tube and 10 mg of DS were dissolved therein. The solution was mixed by vortexing the tube for 5s and the tube was shaken until the DS was dissolved.

**Preparation of PEG400 stock solution 1 mg/mL DS**

All liquids and the DS were equilibrated to ≥ 20°C. An amount of 10 ml of PEG 400 was added to a 50 mL tube and 10 mg of DS were dissolved therein. The solution was mixed by vortexing the tube for 5s and the tube was shaken until the DS was dissolved.

**Preparation ophthalmic formulations 0.05mg/mL DS**

An amount of 2.0mL of the respective stock solution was diluted with 38.0mL of the respective artificial tears solution (either GenTeal® Tears, or Thera® Tears, or Visine® Tears) and the solution was mixed by vortexing for 5s or by stirring for 2min.

**Filter testing for all syringe filters**

The testing was repeated to have samples in triplicate (n=3). An amount of 6 mL of the ophthalmic formulation was withdrawn with a 10mL syringe. An amount of 1 mL was used to determine the final concentration before filtration. The syringe was connected with the filter via Luer Lok and the solution was filtered. Another sample was taken to determine the final concentration after filtration.

The filter was disconnected from the syringe and an amount of 2mL DMSO was withdrawn with a fresh 10mL syringe. The syringe was again connected to the used filter via Luer Lok and the DMSO was filtered. A sample was taken to determine the final concentration after washing.
This protocol was repeated for all tested filters. The following filter materials were tested: PTFE, PVDF, and RC each having a pore size of 0.2μm.

The stock solution with PEG400 diluted with Thera® Tears showed identical results before and after filtration with the filter material PVDF 0.2μm (syringe filter). For all other formulations the PVDF filter was not suitable. The PVDF filter was only suitable for one of 6 formulations.

The PFTE filter was suitable for all formulations diluted with GenTeal® and Visine® Tears.

The RC filter was suitable for all formulations containing any artificial tears solution and for PEG200 as well as for PEG400 stock solution.

**Example 5**

To study the in-use stability with two grades of PEG and 3 different compositions of artificial tears over 5 weeks at room temperature (RT) and 2°C - 8°C in combination with the use of an RC filter material for sterile filtration, a stock solution in PEG with a concentration of 1.0mg/mL of Pritelivir free base hemihydrate was prepared using either PEG200 or PEG400. The stock solutions were diluted in 3 different compositions of artificial tears at a concentration of 0.05mg/mL and filtered through a suitable sterile filter.

| Experiment | DS | Excipients | Scope |
|---|---|---|---|
| 5 | Pritelivir hemihydrate | • PEG200<br>• PEG400<br>• GenTeal® Tears<br>• Thera® Tears<br>• Visine® Tears | A stock solution in PEG with a concentration 1.0mg/mL of DS was prepared by using two different grades of PEG (PEG200 and PEG400). The different stock solutions were diluted in 3 different brands of artificial tears solutions (0.05mg/mL DS) and filtrated through a suitable sterile filter. |

**Preparation of PEG200 stock solution 1 mg/mL DS**

All liquids and the DS were equilibrated to ≥ 20°C. An amount of 20 ml of PEG 200 was added to a 50 mL tube and 20 mg of DS were dissolved therein. The solution was mixed by vortexing the tube for 5s and the tube was shaken until the DS was dissolved.

**Preparation of PEG400 stock solution 1 mg/mL DS**

All liquids and the DS were equilibrated to ≥ 20°C. An amount of 20 ml of PEG 400 was added to a 50 mL tube and 20 mg of DS were dissolved therein. The solution was mixed by vortexing the tube for 5s and the tube was shaken until the DS was dissolved.

**Preparation of ophthalmic formulations 0.05mg/mL**

The respective stock solutions were diluted with artificial tears solution in a beaker.

| Artifical Tears solution | Volume stock solution [mL] | Volume artifical tears [mL] |
|---|---|---|
| GenTeal® Tears | 8.00 | 152.00 |
| Thera® Tears | 2.50 | 47.50 |
| Visine® Tears | 8.00 | 152.00 |

A sample was taken from each beaker on the top, in the middle and on the bottom to determine the final concentration.

**Sterile Filtration**

An amount of 50 mL of the ophthalmic formulation was withdrawn with a 50mL syringe. The syringe was then connected to the respective filter via Luer Lok and the solution was filtered into a beaker. This step was repeated as often as needed by using the same filter and the same syringe for the corresponding batch number and the solution was then refilled into the same beaker. A sample was taken to determine the final concentration after filtration. Three empty 50 mL tubes and one 5 mL tube were provided and the content of one beaker was withdrawn. Amounts of 15mL of the prepared formulation were added to each of the three empty 50mL tube and 5mL to the empty 5mL tube. The samples were stored at room temperature and at a temperature in the range of rom 2°C-8°C over 5 weeks. Only the ophthalmic formulation prepared by using a 1mg/mL stock solution with PEG200 and diluted with GenTeal® Tears could be evaluated. In all other formulations precipitation occurred which probably started before the filtration step using a PTFE and RC filter each having a pore size of 0.2μm.

**Example 6**

To further investigate the stability of the ophthalmic formulation, Pritelivir free base hemihydrate was provided as a 1mg/mL stock solution in PEG400 and diluted with GenTeal® Tears.

| Experiment | DS | Excipients | Scope |
|---|---|---|---|
| 6 | Pritelivir hemihydrate | • PEG400<br>• GenTeal® Tears | The results of experiment 5 were not satisfying so that stability of the ophthalmic formulation was further investigated. |

**Preparation of 1 mg/mL stock solution PEG400**

All liquids and the DS were equilibrated to ≥ 20°C. An amount of 20 ml of PEG 400 was added to a 50 mL tube and 20 mg of DS were dissolved therein. The solution was mixed by vortexing the tube for 5s and the tube was shaken until the DS was dissolved.

**Preparation ophthalmic formulations 0.05mg/mL**

All samples were prepared six fold. Ophthalmic formulations were prepared from the 1mg/mL Pritelivir free base hemihydrate stock solution in PEG400 and GenTeal® Tears in a beaker:

| Artifical Tears | Volume stock solution [mL] | Volume artifical tears [mL] |
|---|---|---|
| GenTeal® Tears | 0.5 | 9.5 |

A sample was taken from each beaker on the top, in the middle and on the bottom to determine the final concentration.

**Sterile Filtration**

The ophthalmic formulation was withdrawn with a 20mL syringe. Then the syringe was connected to an RC 0.2μm filter via Luer Lok and the solution was filtered into a tube. A sample was taken to determine the final concentration after filtration and the samples were stored at room temperature and at a temperature in the range of from 2°C-8°C over 5 weeks.

Six samples in total were prepared in sequence. It took about 5 minutes per sample. All formulations showed comparable assay content before and after filtration. Thus, the preparation time is a critical process parameter. After 1 week the samples stored at room temperature showed an increase of impurities. Samples which were stored at a temperature in the range of from 2-8°C were stable over 1 week.

**Example 7**

To study the impact of the pH value on the tendency of the ophthalmic formulation to degrade and precipitate 3 different compositions of artificial tears were adjusted to pH 4.0, 5.0 and 6.0. A stock solution of Pritelivir free base hemihydrate in PEG400 at a concentration of 1.0mg/mL was prepared. The stock solutions were diluted in the pH adjusted artificial tears at a Pritelivir free base hemihydrate concentration of 0.05mg/mL and filtered through a RC 0.2μm sterile filter. It turned out to be preferable to carry out the dilution step for each sample one after the other to keep the time before the filtration step short.

| Experiment | DS | Excipients | Scope |
|---|---|---|---|
| 7 | Pritelivir hemihydrate | • PEG400<br>• GenTeal® Tears<br>• Thera® Tears<br>• Visine® Tears<br>• 0.1M HCl | The 3 different brands of artificial tears were adjusted to pH 4.0, 5.0 and 6.0.<br>A stock solution in PEG400 with a concentration 1.0mg/mL of DS was prepared. The stock solutions were diluted in the pH adjusted artificial tears solution (0.05mg/mL DS) and filtrated through a RC 0.2μm sterile filter. |

**Preparation of 1 mg/mL stock solution PEG400**

All liquids and the DS were equilibrated to ≥ 20°C. An amount of 40 ml of PEG 400 was added to a 50 mL tube and 40 mg of DS were dissolved therein. The solution was mixed by vortexing the tube for 5s and the tube was shaken until the DS was dissolved.

**Preparation of pH adjusted artificial tears solution**

The following pH values 4.0, 5.0 and 6.0 were adjusted by adding respective amounts of hydrochloric acid to the artificial tears solution.

**Preparation ophthalmic formulations 0.05mg/mL**

All samples were prepared as triplicates. One sample was prepared after the other to keep the preparation time short and to minimize the risk of precipitation. The stock solution was diluted with pH adjusted artificial tears solution in a tube and gently mixed. A sample was taken to determine the final concentration. An amount of 20mL of the tube was withdrawn with a 20mL syringe. Then, the syringe was connected to the RC 0.2mm filter via Luer Lok and the solution was filtered in two portions of 10mL each into two 50mL tube. Another sample was taken to determine the final concentration after filtration.

| Volume stock solution [mL] | Volume artifical tears [mL] |
|---|---|
| 1.0 | 19.0 |

The samples were stored at room temperature and at a temperature in the range of from 2°C-8°C over 4 weeks.

None of the formulations showed a significant change in the pH level over 4 weeks. A higher risk for precipitation was observed for formulations with a higher pH value. The most stable ophthalmic formulation was Visine® Tears adjusted to pH 4. The formulation was stable over 3 weeks stored at room temperature and at a temperature in the range of from 2°C-8°C.

**Example 8**

To determine an acceptable pH value for ophthalmic use, a stock solution of Pritelivir free base hemihydrate in PEG400 was prepared at a concentration 1.0mg/mL. The stock solution was diluted in Visine® Tears solution to a concentration of 0.05mg/mL Pritelivir free base hemihydrate. The ophthalmic formulation was adjusted to pH 6.0, 6.5 and 7.0 and afterwards filtered through a RC 0.2μm sterile filter.

| Experiment | DS | Excipients | Scope |
|---|---|---|---|
| 8 | Pritelivir hemihydrate | • PEG400<br>• Visine® Tears<br>• 0.1M HCl | A stock solution in PEG400 with a concentration 1.0mg/mL of DS was prepared. The stock solution was diluted in Visine® Tears solution (0.05mg/mL DS). The final ophthalmic formulation was adjusted to pH 6.0, 6.5 and 7.0 and afterwards filtrated through a RC 0.2μm sterile filter. |

**Preparation of 1 mg/mL stock solution PEG400**

All liquids and the DS were equilibrated to ≥ 20°C. An amount of 20 ml of PEG 400 was added to a 50 mL tube and 20 mg of DS were dissolved therein. The solution was mixed by vortexing the tube for 5s and the tube was shaken until the DS was dissolved.

**Pre-testing for pH adjustment**

The stock solution was diluted with Visine® tears in a 50mL tube and the resulting solution was mixed gently.

| Volume stock solution [mL] | Volume artifical tears [mL] |
|---|---|
| 0.4 | 7.6 |

A suitable amount of hydrochloric acid was added to adjust pH values of 6.0, 6.5 and 7.0. The resulting solutions were mixed gently.

**Preparation ophthalmic formulations 0.05mg/mL**

All samples were prepared six fold. One sample was prepared after the other to keep the preparation time short and to minimize the risk of precipitation. The stock solution was diluted with Visine® tears solution in a 50mL tube and the resulting solution was mixed gently.

| Volume stock solution [mL] | Volume artifical tears [mL] |
|---|---|
| 0.4 | 7.6 |

The amount of hydrochloric acid was added as determined in the pre-testing to adjust the pH to 6.0 and a sample was taken to determine the final concentration before filtration. The entire content of one tube was withdrawn with a 20mL syringe. Then the syringe was connected to the RC 0.2μm filter via Luer Lok and the solution was filtered into a 50mL tube. A sample was taken to determine the final concentration after filtration of each tube. In addition, samples with corresponding pH values of 6.5 and 7.0 were prepared. All samples were stored at room temperature and at a temperature in the range of from 2°C-8°C over 2 weeks.

A higher risk for precipitation was observed for formulations with a higher pH value. The lower the pH value, the better the stability over two weeks. No significant change of the pH value was observed for all samples over two weeks. The most stable ophthalmic formulation was the ophthalmic formulation adjusted to pH 6.0. The formulation was stable over 1 week stored at room temperature. The ophthalmic formulation adjusted to pH 6.5 was almost as stable as the ophthalmic formulation adjusted to pH 6.0. The ophthalmic formulation adjusted to pH 7.0 showed decreased stability after one week.

**Comparative Example 1**

To study whether Pritelivir free base hemihydrate can be replaced with Pritelivir mesylate, Experiment 8 was repeated with Pritelivir mesylate monohydrate as DS.

| Experiment | DS | Excipients | Scope |
|---|---|---|---|
| 9 | Pritelivir mesylate | • PEG400<br>• Visine® Tears<br>• 0.1M HCl | Ophthalmic formulation was prepared by using Pritelivir mesylate monohydrate as drug substance (DS). |

The preparation protocol of Example 8 was followed also for the ophthalmic formulation containing Pritelivir mesylate monohydrate as drug substance.

However, it was surprisingly found that Pritelvir mesylate monohydrate was not soluble in PEG400 at a concentration of 1 mg/mL. Precipitation was observed and the assay level decreased significantly after filtration.

**Comparative Example 2**

To study whether Pritelivir free base hemihydrate can be replaced with Pritelivir maleate, Experiment 8 was repeated with Pritelivir maleate as DS.

| Experiment | DS | Excipients | Scope |
|---|---|---|---|
| 10 | Pritelivir maleate | • PEG400<br>• Visine® Tears<br>• 0.1M HCl | Ophthalmic formulation was prepared by using Pritelivir maleate as drug substance (DS). |

However, it was surprisingly found that it was not possible to prepare a 1mg/mL stock solution.

**Conclusion:**

From the three tested drug substances only the Pritelivir hemihydrate was suitable for the preparation of an ophthalmic formulation. The above discussed experiments 1 - 10 suggest that the preferred ophthalmic formulation is obtained from a 1mg/mL PEG 400 stock solution which was diluted with Visine® Tears solution (also named Visine® Tears) and adjusted to pH 6.0.

**Examples of ophthalmic formulations**

**Formulation 1:**

Prepared according to Example 8
Stock solution: 1 mg Pritelivir free base hemihydrate in 1 ml PEG 400.
Ophthalmic formulation:
0.4 ml stock solution and 7.6 ml Visine® Tears solution to obtain 8.0 ml ophthalmic formulation. pH was adjusted to 6.0.

**Formulation 2:**

Prepared according to Example 8.
Stock solution: 1 mg Pritelivir free base hemihydrate in 1 ml PEG 400.
Ophthalmic formulation:
0.4 ml stock solution and 7.6 ml Visine® Tears solution to obtain 8.0 ml ophthalmic formulation. pH was adjusted to 6.5.

**Formulation 3:**

Prepared according to Example 8.
Stock solution: 1 mg Pritelivir free base hemihydrate in 1 ml PEG 400.
Ophthalmic formulation:
0.4 ml stock solution and 7.6 ml Visine® Tears solution to obtain 8.0 ml ophthalmic formulation. pH was adjusted to 7.0.

**Formulation 4:**

Prepared in line with Example 5.
Stock solution: 1 mg Pritelivir free base hemihydrate in 1 ml PEG 200.
Ophthalmic formulation:
8 ml stock solution and 152 ml GenTeal® Tears solution to obtain 160.0 ml ophthalmic formulation. In addition, the pH was adjusted to 6.0.

**Formulation 5:**

Prepared in line with Example 5.
Stock solution: 1 mg Pritelivir free base hemihydrate in 1 ml PEG 200.
Ophthalmic formulation:
2.5 ml stock solution and 47.5 ml GenTeal® Tears solution to obtain 50.0 ml ophthalmic formulation. In addition, the pH was adjusted to 6.8.

**Formulation 6:**

Prepared in line with Example 4.
Stock solution: 1 mg Pritelivir free base hemihydrate in 0.5 ml PEG 200 and 0.5 ml PEG400.
Ophthalmic formulation:
8 ml stock solution and 152 ml Thera® Tears solution to obtain 160.0 ml ophthalmic formulation. In addition, the pH was adjusted to 7.0.

**Formulation 7:**

Stock solution: 1.5 mg Pritelivir free base hemihydrate in 1 ml PEG 300.
Artificial tears solution: 2.5 g/L glycerin, 2 g/L hypromellose, and 11.28 g/L polyethylene glycol 400 in purified water containing polyvinyl alcohol, povidone, propylene glycol, ascorbic acid, magnesium chloride, potassium chloride, sodium borate, sodium chloride, sodium citrate, and/or sodium lactate.
Ophthalmic formulation:
1 ml stock solution and 15 ml artificial tears solution to obtain 16.0 ml ophthalmic formulation. pH was adjusted to 6.5.

## Claims

1. An ophthalmic formulation comprising:

   a) N-[5-(aminosulfonyl)-4-methyl-1,3-thiazol-2-yl]-N-methyl-2-[4-(2-pyridinyl)phenyl]acetamide hemihydrate,
   b) polyethylene glycol, and
   c) artificial tears solution.

2. The ophthalmic formulation according to claim 1, wherein the ophthalmic formulation has a pH value in the range of from 4.0 to 7.0.

3. The ophthalmic formulation according to claim 1 or 2, wherein the ophthalmic formulation has a pH value in the range of from 5.5 to 6.5.

4. The ophthalmic formulation according to any one of the claims 1 to 3, wherein the ophthalmic formulation has a viscosity between 25 cps and 50 cps.

5. The ophthalmic formulation according to any one of the claims 1 to 4, wherein the polyethylene glycol has an average molecular weight in the range of from 200 g/mol to 400 g/mol.

6. The ophthalmic formulation according to any one of the claims 1 to 5, wherein the polyethylene glycol is contained in the ophthalmic formulation in an amount in the range of from 40 μg per ml ophthalmic formulation to 70 μg per ml ophthalmic formulation.

7. The ophthalmic formulation according to any one of the claims 1 to 6, wherein the N-[5-(aminosulfonyl)-4-methyl-1,3-thiazol-2-yl]-N-methyl-2-[4-(2-pyridinyl)-phenyl]acetamide hemihydrate is contained in the ophthalmic formulation in an amount in the range of from 40 μg per ml ophthalmic formulation to 60 μg per ml ophthalmic formulation.

8. The ophthalmic formulation according to any one of the claims 1 to 7, wherein the artificial tears solution is contained in the ophthalmic formulation in an amount in the range of from 0.9 g per ml ophthalmic formulation to 1.3 g per ml ophthalmic formulation.

9. The ophthalmic formulation according to any one of the claims 1 to 8, wherein the artificial tears solution contains glycerin, hypromellose, and polyethylene glycol 400 and optionally povidone.

10. The ophthalmic formulation according to claim 9, wherein the wherein the artificial tears solution further contains carboxymethylcellulose, dextran, polysorbate, polyvinyl alcohol, povidone, propylene glycol, ascorbic acid, benzalkonium chloride, boric acid, dextrose, disodium phosphate, glycine, magnesium chloride, potassium chloride, purified water, sodium borate, sodium chloride, sodium citrate, and/or sodium lactate.

11. The ophthalmic formulation according to any one of the claims 1 to 8, wherein the artificial tears solution is Visine® tears solution.

12. A method for the production of the ophthalmic formulation as defined in claim 1, the method comprising the steps

    I) providing N-[5-(aminosulfonyl)-4-methyl-1,3-thiazol-2-yl]-N-methyl-2-[4-(2-pyridinyl)phenyl]acetamide hemihydrate,
    II) providing polyethylene glycol,

III) providing artificial tears solution,
IV) preparing a stock solution of N-[5-(aminosulfonyl)-4-methyl-1,3-thiazol-2-yl]-N-methyl-2-[4-(2-pyridinyl)phenyl]acetamide hemihydrate in polyethylene glycol,
V) diluting the stock solution with artificial tears solution until the desired concentration is reached,
VI) adjusting the pH value by addition of an aqueous solution of an acid to the desired pH value, and
VII) performing a sterial filtration by means of a suitable sterile filter to obtain the ophthalmic formulation.

**13.** The method according to claim 12, wherein the pH value is adjusted to a value in the range of from 5.5 to 6.5.

**14.** The method according to claim 12 or 13, wherein the sterile filtration is performed with a regenerated cellulose (RC) 0.2 μm filter.

**15.** The method according to any one of claims 12 to 14, wherein the polyethylene glycol is polyethylene glycol 400 and/or the artificial tears solution is the Visine® tears solution.

Europäisches Patentamt
European Patent Office
Office européen des brevets

# EUROPEAN SEARCH REPORT

Application Number
EP 20 18 0465

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2018/095576 A1 (AICURIS ANTI INFECTIVE CURES GMBH [DE]) 31 May 2018 (2018-05-31) * p.15 l.4-p.36 last line, p.39 l.3-p.42 l.9, p.65 l.25-p.66 l.2; claims * ----- | 1-15 | INV. A61K9/00 |
| X | WO 2018/096170 A1 (AICURIS ANTI INFECTIVE CURES GMBH [DE]) 31 May 2018 (2018-05-31) * p.18 l.14-19 and p.43 l.4-14; claim claims * ----- | 1-3,5, 7-10 | |
| A | US 2004/235917 A1 (BETZ ULRICH [DE] ET AL) 25 November 2004 (2004-11-25) * paragraph [0265] - paragraph [0273]; claims; examples 1-4; table 4 * ----- | 1-15 | |

TECHNICAL FIELDS SEARCHED (IPC)
A61K

The present search report has been drawn up for all claims

1

| Place of search | Date of completion of the search | Examiner |
|---|---|---|
| The Hague | 1 December 2020 | Couckuyt, Philippe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 20 18 0465

This annex lists the patent family members relating to the patent documents cited in the above-mentioned European search report. The members are as contained in the European Patent Office EDP file on The European Patent Office is in no way liable for these particulars which are merely given for the purpose of information.

01-12-2020

| Patent document cited in search report | Publication date | Patent family member(s) | Publication date |
|---|---|---|---|
| WO 2018095576 A1 | 31-05-2018 | AR 110250 A1 | 13-03-2019 |
| | | TW 201825095 A | 16-07-2018 |
| | | UY 37497 A | 29-06-2018 |
| | | WO 2018095576 A1 | 31-05-2018 |
| WO 2018096170 A1 | 31-05-2018 | AR 110249 A1 | 13-03-2019 |
| | | AU 2017365632 A1 | 13-06-2019 |
| | | CA 3045059 A1 | 31-05-2018 |
| | | CN 109996798 A | 09-07-2019 |
| | | EP 3544976 A1 | 02-10-2019 |
| | | JP 2020509991 A | 02-04-2020 |
| | | KR 20190085115 A | 17-07-2019 |
| | | TW 201825483 A | 16-07-2018 |
| | | US 2020123145 A1 | 23-04-2020 |
| | | UY 37496 A | 29-06-2018 |
| | | WO 2018096170 A1 | 31-05-2018 |
| US 2004235917 A1 | 25-11-2004 | AT 320253 T | 15-04-2006 |
| | | AT 464047 T | 15-04-2010 |
| | | BR 0210601 A | 28-09-2004 |
| | | CA 2451552 A1 | 03-01-2003 |
| | | CN 1535149 A | 06-10-2004 |
| | | DE 10129714 A1 | 02-01-2003 |
| | | DK 1401435 T3 | 17-07-2006 |
| | | DK 1629842 T3 | 02-08-2010 |
| | | EP 1401435 A1 | 31-03-2004 |
| | | EP 1629842 A1 | 01-03-2006 |
| | | ES 2260449 T3 | 01-11-2006 |
| | | ES 2341778 T3 | 28-06-2010 |
| | | HK 1069986 A1 | 10-06-2005 |
| | | JP 4342304 B2 | 14-10-2009 |
| | | JP 2004534818 A | 18-11-2004 |
| | | KR 20040030689 A | 09-04-2004 |
| | | MX PA03011518 A | 28-10-2004 |
| | | PL 364378 A1 | 13-12-2004 |
| | | PT 1401435 E | 31-07-2006 |
| | | PT 1629842 E | 10-05-2010 |
| | | US 2004235917 A1 | 25-11-2004 |
| | | WO 03000259 A1 | 03-01-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0147904 A1
- WO 2006103011 A1